(19) **Europäisches Patentamt**

**European Patent Office**

**Office européen des brevets**

(11) **EP 1 375 635 A1**

(12) **EUROPEAN PATENT APPLICATION**
published in accordance with Art. 158(3) EPC

(43) Date of publication:
**02.01.2004 Bulletin 2004/01**

(51) Int Cl.⁷: **C11D 7/54**, C11D 7/20, A61K 7/20

(21) Application number: **02701596.5**

(22) Date of filing: **27.02.2002**

(86) International application number:
**PCT/JP2002/001799**

(87) International publication number:
**WO 2002/068576 (06.09.2002 Gazette 2002/36)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.02.2001 JP 2001052682**

(71) Applicant: **Ecodevice Laboratory Co., Ltd Iruma-shi, Saitama 358-0026 (JP)**

(72) Inventors:
- **SUGIHARA, Shinichi Yokohama-shi, Kanagawa 227-0052 (JP)**
- **KAWABE, Kenji Ogasa-gun, Shizuoka 439-0019 (JP)**

(74) Representative: **Patentanwälte Ruff, Wilhelm, Beier, Dauster & Partner Kronenstrasse 30 70174 Stuttgart (DE)**

(54) **BLEACHING COMPOSITION AND METHOD OF BLEACHING TOOTH**

(57) A bleaching composition comprising a peroxide and a photocatalyst (a visible light responsive photocatalyst) activated by the effect of light having a wavelength of greater than or equal to 420 nm. A method of bleaching teeth comprising application of the composition according to any of claims 1 to 4 on the surface of a tooth and irradiating the resulting coating with light comprising a component with a wavelength of greater than or equal to 420 nm.

## Description

### Technical Field

The present invention relates to a bleaching composition comprising a visible-light responsive photocatalyst as well as to a method of bleaching teeth employing this bleaching composition.

### Technical Background

White teeth are desired not just by entertainers, but also by the general public. There is a particularly strong desire among the young to have white teeth. Accordingly, the bleaching of teeth has gradually become widespread in dental diagnosis. The bleaching of teeth comprises highly safe home bleaching that is conducted at home, and office bleaching, which affords a considerable bleaching effect and is conducted by a dentist in consideration of the discoloration of a patient's teeth and the cause of the discoloration. In contrast to home bleaching, in which compounds such as hydrogen peroxide are employed at relatively low concentrations, office bleaching generally involves the use of compounds such as hydrogen peroxide at relatively high concentrations combined with argon lasers and photopolymerization devices.

Since compounds such as hydrogen peroxide have been employed in this manner at relatively high concentrations in office bleaching, contact with the compounds has caused the gums to become inflamed and contract during the bleaching process. Although the teeth can be bleached, there is a problem in that the gums are stripped back and deteriorate. Thus, in office bleaching, the gums are generally treated prior to bleaching to protect them. However, there is a problem in that the time and cost required to protectively treat the gums become necessary extras.

Although there are a variety of causes behind dental discoloration, there is a problem in that tetracycline discoloration (discoloration due to the use of drugs in the tetracycline family) is aggravated by exposure to ultraviolet radiation. Accordingly, although visible light is often employed in bleaching, the use of visible light requires long periods of treatment. With long periods of treatment, there is a problem in that gum inflammation sometimes occurs even when the gums have been protectively treated. Further, in the case of tetracycline discoloration, bleaching to desired levels is sometimes impossible using conventional bleaching methods.

Accordingly, there is a strong need for a new dental bleaching method that is safe, convenient, and capable of rapidly achieving the desired effect. In this context, Japanese Unexamined Patent Publication (KOKAI) Heisei No. 11-92351 provides a bleaching method employing as active components both a hydrogen peroxide aqueous solution and titanium dioxide, which has a photocatalytic effect; a bleaching agent employing this bleaching method; a method of manufacturing the same; and a tooth bleaching system employing this bleaching agent.

The inventions described in Japanese Unexamined Patent Publication (KOKAI) Heisei NO. 11-92351 are as follows:

(1) A discolored tooth bleaching method characterized by adhering a solution/paste of titanium dioxide powder and hydrogen peroxide aqueous solution to the surface of a discolored tooth and irradiating this portion with light to produce a photocatalytic effect, thereby bleaching the tooth.

(2) The discolored tooth bleaching method of (1) above further characterized in that violet visible light is irradiated.

(3) A bleaching agent that is adhered to the surface of a discolored tooth with that portion then being irradiated with light to produce a photocatalytic reaction to bleach the discolored tooth, characterized in that a hydrogen peroxide aqueous solution and titanium dioxide undergoing a photocatalytic reaction when irradiated with light are employed in combination as active components.

(4) The bleaching agent of (3) above further characterized by being comprised of an aqueous solution/paste containing titanium dioxide with a particle diameter of about 5 to 60 nm and a less than or equal to 3 percent hydrogen peroxide aqueous solution.

(5) A method of manufacturing a bleaching agent that is adhered to the surface of a discolored tooth with that portion then being irradiated with light to produce a photocatalytic reaction to bleach the discolored tooth, characterized in that titanium dioxide powder producing a photocatalytic reaction on hydrogen peroxide aqueous solution when irradiated with light is blended in.

(6) The method of manufacturing a bleaching agent of (5) above further characterized in that titanium dioxide powder producing a photocatalytic reaction on an aqueous solution of less than or equal to 3 percent hydrogen peroxide is blended in.

(7) The method of manufacturing a bleaching agent of (5) or (6) above further characterized in that anatase titanium dioxide is blended in as the titanium dioxide powder.

(8) A dental bleaching system combining the bleaching agent of (3) or (4) above with a means of adhering said bleaching agent, an irradiating device, and/or some other tooth treatment material.

(9) The tooth bleaching system of (8) above characterized by further comprising an irradiating device emitting visible violet light.

The titanium dioxide employed in the above-described bleaching agent is anatase titanium dioxide. Anatase titanium dioxide normally requires ultraviolet radiation of less than or equal to 400 nm as excitation radiation. Some anatase and rutile titanium dioxides comprised of microparticles exhibit activity even in the visible light range, but this is true at most for light of less than or equal to 410 nm and the effect is minimal. Accordingly, although visible light is employed in the above-described bleaching methods, it is irradiated in the form of violet light, with only light in an extremely limited range in the vicinity of 400 nm being employed within the visible light range of 400 to 700 nm.

As a result, the treatment still requires a period of at least one hour, and even when the concentration of hydrogen peroxide employed is low, the negative effects on blood and gums during the treatment period cannot be avoided. Since it is necessary to continue the irradiation of light during the bleaching treatment, patients undergoing bleaching treatment in which a light irradiating device is installed are subjected to considerable pain.

Japanese Unexamined Patent Publication (KOKAI) No. 2000-344640 discloses a bleaching agent for home bleaching as follows:

(1) A bleaching agent for home bleaching comprised of a titanium dioxide sol into which are blended ultrafine titanium dioxide particles coated with calcium phosphate, exhibiting the effect of adsorbing and eliminating the organic components causing the discoloration of teeth.

(2) The bleaching agent for home bleaching of (1) above wherein ultrafine titanium dioxide particles that have been obtained by coating a titanium hydroxide sol -- obtained by dispersing ultrafine titanium dioxide particles in water -- with calcium phosphate are blended in.

(3) The bleaching agent for home bleaching of (1) or (2) above wherein an inorganic layered compound is blended into the titanium dioxide sol.

(4) The bleaching agent for home bleaching of any of (1) to (3) above wherein the titanium dioxide content is less than or equal to 0.999 weight percent.

(5) The bleaching agent for home bleaching of any of (1) to (4) above wherein dissolved oxygen is present in the bleaching agent for home bleaching.

(6) A bleaching agent kit for home bleaching comprising any of the bleaching agents for home bleaching of (1) to (5) above, a light source causing a photocatalytic reaction, and as needed, a suitable treatment means.

(7) The bleaching agent kit for home bleaching of (6) above wherein a visible light radiating device such as a visible light LED lamp or visible light semiconductor laser radiating device is employed as the light source causing a photocatalytic reaction.

The above-cited patent publication states that teeth can be bleached by irradiation with visible light when the above-described bleaching agents for home bleaching are employed. Although a photocatalyst with visible light activity is a basic requirement for bleaching with visible light, there is no description of titanium oxide having visible light activity in Japanese Unexamined Patent Publication (KOKAI) No. 2000-344640. The titanium oxide employed in the invention described in Japanese Unexamined Patent Publication (KOKAI) No. 2000-344640 is a photocatalyst reacting primarily to ordinary ultraviolet radiation. Thus, a bleaching time of several hours is described in the examples.

Accordingly, a period of greater than or equal to one hour is still required for treatment, and the treatment period is still long even when hydrogen peroxide is employed at low concentrations, making it impossible to avoid a negative effect on the blood and gums. Further, since irradiation with light must be continued during the bleaching treatment, patients subjected to a bleaching treatment in which a light irradiating device is installed must endure considerable pain.

Accordingly, the object of the present invention is to provide a new bleaching agent and a new bleaching method that employ photocatalysts and are capable of producing a desired effect in a shorter time than in prior art.

## Disclosure of the Invention

The present invention relates to a bleaching composition comprising a peroxide and a photocatalyst (referred to hereinafter as a visible light responsive photocatalyst) activated by the effect of light having a wavelength of greater than or equal to 420 nm.

The following are preferred modes of the bleaching composition of the present invention:

(1) A visible light responsive photocatalyst that is activated by light having a wavelength of greater than or equal to 450 nm.

(2) A visible light responsive photocatalyst that is a visible light responsive material in the form of titanium oxide comprising at least anatase titanium oxide, where in ESR measurement under irradiation by light having a wavelength of greater than or equal to 420 nm at 77 K under vacuum, a primary signal with a g value of from 2.004 to 2.007 and two secondary signals with g values of from 1.985 to 1.986 and 2.024 are detected, and these three signals are either only slightly detected or essentially undetected under vacuum at 77 K in the dark.

(3) The peroxide is hydrogen peroxide or urea peroxide.

(4) The bleaching composition is employed to bleach teeth.

Further, the present invention relates to a method of bleaching teeth comprising the application of the composition of the present invention on the surface of a tooth and irradiating the coating with light comprising a component with a wavelength of greater than or equal to 420 nm.

The following are preferred modes of the bleaching method of the present invention.

(1) The light comprising a component with a wavelength of greater than or equal to 420 nm is plasma light or light generated by a photopolymerization device, light-emitting diode, or halogen lamp.

(2) The light-emitting diode is a violet light-emitting diode, blue light-emitting diode, green light-emitting diode, yellow light-emitting diode, or white light-emitting diode.

### Brief Description of the Drawings

Fig. 1 shows ESR spectra of the visible light responsive type material of the present invention (Reference Example 1) measured at 77 K in vacuum. The upper segment is a spectrum measured in darkness, the middle segment is a spectrum measured with irradiation by light (light below 420 nm was cut off in light from a mercury lamp) having a wavelength of not less than 420 nm, and the lower segment is a spectrum measured with irradiation with light from a mercury lamp from which light of less than 420 nm was not cut off.

Fig. 2 shows ESR spectra of the visible light responsive type material of the present invention (Reference Example 1) measured at ordinary temperature in vacuum. The upper segment is a spectrum measured in darkness, the middle segment is a spectrum measured with irradiation by light (light below 420 nm was cut off in light from a mercury lamp) having a wavelength of not less than 420 nm, and the lower segment is a spectrum measured with irradiation with light from a mercury lamp from which light of less than 420 nm was not cut off.

Fig. 3 gives the results of measurement by XRD of the product of Embodiment 1 (upper segment) and a hydrolysis product (dried at 50°C) (lower segment).

Fig. 4 shows light-emitting spectra of a blue light-emitting diode (NSPB), green light-emitting diode (NSPG) and white light-emitting diode (NSPW).

Fig. 5 shows ESR spectra of Reference Example 4 (the visible light responsive type material) measured at 77 K in vacuum with irradiation by light (light below 420 nm was cut off in light from a mercury lamp) having a wavelength of not less than 420 nm.

Fig. 6 shows ESR spectra of Reference Example 5 (the visible light responsive type material) measured at 77 K in vacuum with irradiation by light (light below 420 nm was cut off in light from a mercury lamp) having a wavelength of not less than 420 nm.

Fig. 7 shows ESR spectra of Reference Example 6 (the visible light responsive type material) measured at 77 K in vacuum with irradiation by light (light below 420 nm was cut off in light from a mercury lamp) having a wavelength of not less than 420 nm.

Fig. 8 shows the measured results of the L* color system of apatite colored with tetracycline before and after bleaching.

Fig. 9 shows the measured results of the a* color system of apatite colored with tetracycline before and after bleaching.

Fig. 10 shows the measured results of the b* color system of apatite colored with tetracycline before and after bleaching.

## Best Mode of Implementing the Invention

The visible light responsive type photocatalyst used for the bleaching composition of the present invention may be, for example, a visible light responsive type material in the form of titanium oxide comprising at least anatase titanium oxide, with a main signal (the strongest signal) having a g value ranging from 2.004 to 2.007 and two subsignals (signals of lesser intensity than the main signal) having a g value ranging from 1.985 to 1.986 and a g value at 2.024 as measured by ESR under the irradiation of light having a wavelength of 420 nm or more at 77 K in vacuum. With the visible light responsive type material, the three signals (one main signal and two subsignals) are observed in a little amount or substantially not observed in darkness at 77 K in vacuum.

The light having a wavelength of not less than 420 nm employed in ESR is obtained by passing light from a high-pressure mercury lamp (for example, 500 W) through a filter (L-42) cutting out light with a wavelength below 420 nm.

Further, even in ESR measurement conducted with irradiation by light having a wavelength of not less than 455 nm at 77 K in vacuum (obtained by passing light from a xenon lamp (for example, 150 W) through a filter (GG455) cutting light of wavelengths below 455 nm), the visible light responsive type material sometimes exhibits a main signal (the strongest signal) having a g value ranging from 2.004 to 2.007 and two subsignals (signals of lesser intensity than the main signal) having a g value ranging from 1.985 to 1.986 and a g value at 2.024.

The visible light responsive type material used for the bleaching composition of the present invention may be, for example, a visible light photocatalyst comprised of titanium dioxide having stable oxygen defects described in WO 00/10706. The ESR spectrum of the visible light photocatalyst described in WO 00/10706 has only a signal having a g value ranging from 2.003 to 2.004 in ESR measurement in the dark at 77 K in vacuum. The visible light photocatalyst described in WO 00/10706 is heterogenic with the spectrum exhibited by the above-mentioned visible light responsive type material. However, both exhibit activity with visible light having a wavelength of not less than 420 nm, preferably visible light having a wavelength of not less than 450 nm.

The above-mentioned visible light responsive type material will be set forth below.

The visible light responsive type material used in the present invention is preferably titanium oxide comprising a main component in the form of anatase titanium oxide, and may additionally comprise amorphous titanium oxide. Alternatively, it may comprise rutile titanium oxide. Further, the anatase titanium oxide need not necessarily have a high degree of crystallinity. Still further, the titanium oxide constituting the visible light responsive type material may comprise titanium and oxygen in non-stoichiometric ratio. Specifically, the quantity of oxygen relative to titanium may be lower than the stoichiometric ratio (a theoretic value of 2.00) of titanium dioxide. In the titanium oxide in the visible light responsive type material, the molar ratio of oxygen to titanium may be, for example, less than 2.00, for example, 1.00-1.99, or 1.50-1.99. The molar ratio of oxygen to titanium in the titanium oxide in the visible light responsive type material can be measured, for example, by X-ray photoelectron spectroscopy.

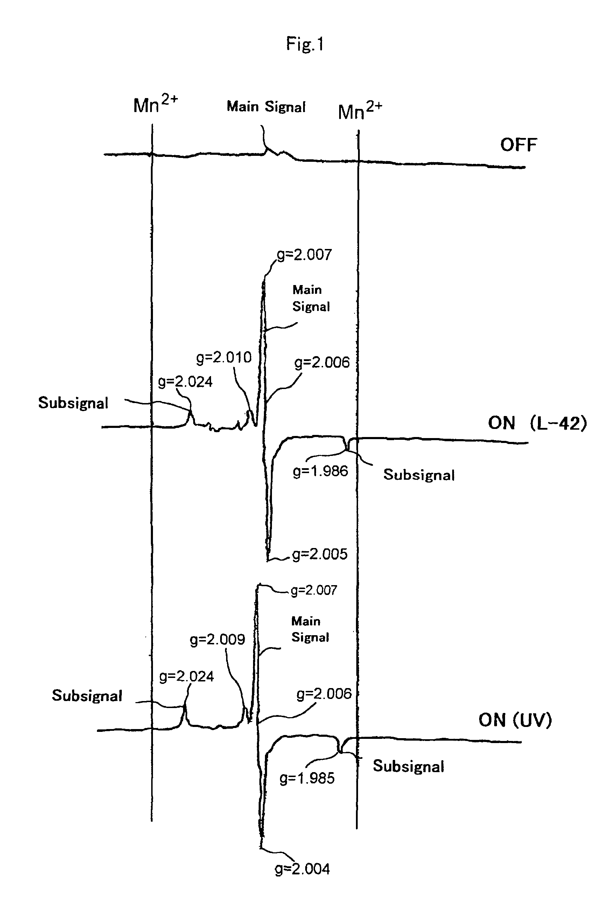

Fig. 1 shows conventional ESR spectra measured at 77 K in vacuum for the above-mentioned visible light responsive type material. In the figure, the upper segment is a spectrum measured in darkness and the middle segment is a spectrum measured with irradiation with light having a wavelength of not less than 420 nm (obtained by cutting off light of less than 420 nm in light from a mercury lamp). The lower segment is a spectrum obtained by irradiation with light from a mercury lamp from which light of less than 420 nm has not been cut off. The upper segment, middle segment, and lower segment are all results obtained at identical GAIN.

In the spectrum of the upper segment of Fig. 1, the main signal having a g value ranging from 2.004-2.007 was measured as being quite small and the two subsignals having g values of 1.985-1.986 and 2.024 were essentially not detected. Further, as is clear from a comparison of the upper segment and middle segment spectra in Fig. 1, in the middle segment spectrum, the main signal having a g value ranging from 2.004 to 2.007 and the two subsignals having g values ranging from 1.985 to 1.986 and 2.024 are substantially much greater in intensity than those of the upper segment spectrum. Further, as is clear from a comparison of the middle segment and lower segment spectra of Fig. 1, the intensity of the main signal having a g value of 2.004 to 2.007 and those of the two subsignals having g values of 1.985 to 1.986 and 2.024 essentially did not differ regardless of whether or not light of less than 420 nm was included in the irradiating light.

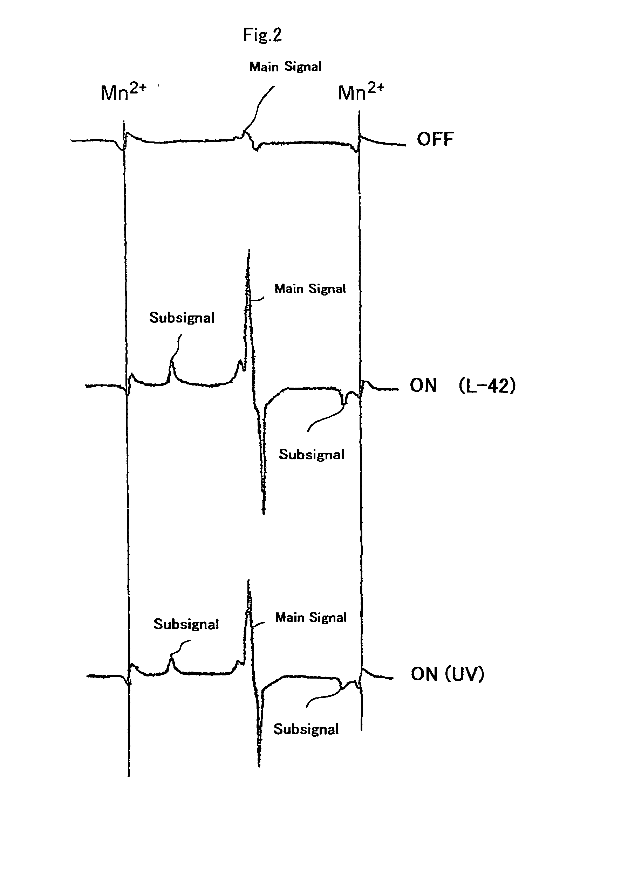

Further, as shown in Fig. 2, the main signal having a g value of 2.004-2.007 was observed to be quite small and the two subsignals having g values ranging from 1.985 to 1.986 and 2.024 were essentially not detected for the visible light responsive type material in darkness at ordinary temperature in vacuum. Further, it was found that the three signals were detected by ESR with irradiation by light having a wavelength of not less than 420 nm and light from a mercury lamp from which light of less than 420 nm had not been cut off at ordinary temperature in vacuum. In Fig. 2, the upper segment is a spectrum measured in darkness and the middle segment is a spectrum measured with irradiation by light having a wavelength of not less than 420 nm (obtained by cutting off light of less than 420 nm in light from a mercury lamp). The lower segment is a spectrum obtained by irradiation with light from a mercury lamp from which light of less than 420 nm was not cut off. The upper, middle, and lower segments are all the results of measurements made at identical GAIN.

In addition to the above-described signals, the visible light responsive type material may also have a subsignal having a g value ranging from 2.009 to 2.010 when measured with irradiation by light having a wavelength of not less than 420 nm at 77 K in vacuum. The subsignal having a g value ranging from 2.009 to 2.010 is shown in the ESR spectrum of the middle segment of Fig. 1.

As set forth above, the visible light responsive type material has a characteristic ESR signal. In addition, it is also a colored material. For example, when the reflectance for light with a wavelength of 600 nm is designated as 1 (or 100 percent), the reflectance for light with a wavelength of 450 nm can be not greater than 0.85 (or 85 percent), preferably not greater than 0.80 (or 80 percent), and still more preferably, not greater than 0.70 (or 70 percent). The greater the coloration, the greater the tendency toward visible light responsive activity. The reflectance referred to here is the result of measurement with a spectrophotometer. Although reflectance can also be measured with a color analyzer, the evaluation of the above-mentioned reflectance is conducted with a photospectrometer for better precision.

As set forth above, the visible light responsive type material has a characteristic ESR signal. In addition, it also has oxidation activity on NO for light in the visible range. Specifically, irradiation with visible light with a wavelength of at least 520 nm, or lower, results in NO oxidation activity. In the preferred material, irradiation with visible light with a wavelength of 570 nm or less results in NO oxidation activity.

The visible light responsive type material can be manufactured from a starting material in the form of amorphous or incomplete crystalline titanium oxide (including hydrous titanium oxide) and/or titanium hydroxide. The titanium compound starting material can be obtained by a wet method such as the sulfuric acid method or chloride method. More specifically, the titanium compound starting material can be obtained by hydrolyzing titanium chloride or titanium sulfate with ammonium hydroxide. Alternatively, the titanium compound starting material can be obtained by hydrolyzing titanium alkoxide with water or hydrolyzing titanium alkoxide with an ammonium hydroxide aqueous solution. However, from the perspective of the low cost of the starting materials, those obtained by hydrolysis of titanium chloride or titanium sulfate with ammonium hydroxide are preferred in industrial production. Accordingly, the hydrolysis of titanium chloride or titanium sulfate with ammonium hydroxide is described below.

The above-mentioned hydrolysis can be conducted, for example, by continuously or intermittently adding ammonium hydroxide aqueous solution to a titanium chloride aqueous solution or titanium sulfate aqueous solution, or continuously or intermittently adding a titanium chloride aqueous solution or titanium sulfate aqueous solution to an ammonium hydroxide aqueous solution. The concentrations of the titanium chloride aqueous solution, titanium sulfate aqueous solution, and ammonium hydroxide aqueous solution can be suitably determined. In the hydrolysis, the amount of ammonium hydroxide added is suitably adjusted to produce an alkaline reaction solution with a final pH of not less than 5. The titanium chloride may be in the form of titanium trichloride, titanium tetrachloride, or the like, or a mixture thereof. The hydrolysis can be conducted, for example, at a temperature of 0-100°C, preferably 20-80°C. However, hydrolysis at ordinary temperature is sometimes preferred from the viewpoint of obtaining titanium dioxide of comparatively low crystallinity or that is amorphous.

The hydrolysis product of titanium chloride or titanium sulfate with ammonium hydroxide is desirably washed with water or an ammonium hydroxide aqueous solution and employed as the starting material titanium compound. The washing of the hydrolysis product with water or ammonium hydroxide aqueous solution, for example, can be conducted by filtering the reaction solution comprising the hydrolysis product and further passing water or ammonium hydroxide aqueous solution through the hydrolysis product obtained as a filtrate. This method is preferred because of easy operation that it suffices to add water or ammonium hydroxide aqueous solution to the filtered hydrolysis product as it is and conduct filtration. In addition to the above, the washing of the hydrolysis product with water or ammonium hydroxide aqueous solution can be conducted by, for example, resuspending the hydrolysis product filtrate in water or ammonium hydroxide aqueous solution and filtering the suspension obtained. Washing with water or ammonium hydroxide aqueous solution is conducted to suitably decrease the quantity of residual ammonium salts such as ammonium chloride or ammonium sulfate produced during hydrolysis, and can be conducted multiple times.

Commercial products of amorphous or incomplete crystalline titanium dioxide may also be employed. Examples are incomplete crystalline titanium dioxide such as ST-01 and C-02 manufactured by Ishihara Sangyo.

In the manufacturing method, the starting material titanium compound such as amorphous or incomplete crystalline titanium oxide is heated in the presence of ammonia or a derivative thereof. The ammonia may be liquid or gaseous. When using ammonia gas, the starting material titanium compound is heated in an ammonia gas atmosphere. Examples of ammonia derivatives are ammonium salts such as ammonium hydroxide and ammonium chloride; the starting material titanium compound is heated, for example, in the presence of ammonium hydroxide or ammonium chloride.

The heating of the starting material titanium compound in the presence of ammonia or a derivative thereof is stopped when the absorbance of light at a wavelength of 450 nm by the material produced by the heating becomes greater than the absorbance of light at a wavelength of 450 nm by the starting material titanium compound. Usually, the starting material titanium compound is white and its absorbance of light at a wavelength of 450 nm is about 10 percent. By contrast, when the starting material titanium compound is heated in the presence of ammonia or a derivative thereof, it gradually turns yellow. However, this coloration peaks at a certain point and then reduces, finally becoming a compound with absorbance about the same as the starting material titanium compound. Although the absorbance of light at a wavelength of 450 nm varies with the type of starting material titanium compound, type and quantity of ammonia (derivative) co-existed, heating temperature and time and the like, it sometimes reaches a maximum of about 60 percent. The characteristic of the visible light responsive type material is not definitively determined based on the intensity of absorbance of light at a wavelength of 450 nm, but when the absorbance of light at a wavelength of 450 nm is 15 percent or greater (reflectance is 85 percent or less), it has clearly become a material exhibiting visible light responsiveness. Accordingly, in the above-described heat treatment, it is desirable to set conditions so that when reflectance of light with a wavelength of 600 nm is defined as 1 (or 100 percent), the reflectance of light with a wavelength of 450 nm is 0.85 (or 85 percent) or less, preferably 0.80 (or 80 percent) or less, and more preferably, 0.70 (or 70 percent) or less. The absorbance referred to here is the result of measurement with a spectrophotometer.

The above-mentioned heating conditions cannot necessarily be specified by just temperature, but the temperature employed falls within a range of 250-550°C, for example. There is a tendency that the $NO_x$ elimination rate due to light with wavelengths of 420 and 470 nm is comparatively high with heating to within a range of 300-450°C, and even higher with heating to within a range of 325-425°C, and the $NO_x$ elimination rate due to light with wavelengths of 520 and 570 nm is comparatively high with heating to within a range of 325-450°C, and even higher with heating to within a range of 350-425°C. Accordingly, from the viewpoint of the high elimination rate of $NO_x$ in the visible light range, heating at a temperature ranging from 350-425°C is of greatest preference.

From the viewpoint of improving the $NO_x$ elimination rate for light within the visible range having wavelengths of 520 nm and 570 nm, a heating time of at least 30 min, preferably at least 1 hr, is desirable. Further, since no significant change in the $NO_x$ elimination rate was observed when the heating time was extended beyond 1 hr, the maximum heating time is about 3 hr.

The heating can be conducted at ordinary pressure. Further, the heating time can be suitably determined based on the absorbance of light with a wavelength of 450 nm by the material produced by heating.

The above-mentioned heating can be performed with one of the rotary kilns, tunnel kilns, muffle furnaces, or the like commonly employed in this field. When the individual grains of titanium oxide aggregate or sinter by heating, they can be comminuted as needed with a pulverizer.

Further, the material obtained by heating as set forth above can be washed with water or an aqueous solution as necessary. This washing sometimes improves the visible light responsiveness of the visible light responsive type material obtained. Further, based on conditions, a material having good visible light responsiveness can sometimes be obtained without washing.

When the amorphous or incomplete crystalline titanium oxide (the starting material titanium compound prior to heating) is those obtained, for example, by hydrolyzing titanium chloride with ammonium hydroxide, a considerable amount of ammonium chloride remains in the hydrolysis product. As a result, heating of the amorphous or incomplete crystalline titanium dioxide to a prescribed temperature as set forth above can convert it to a visible light responsive type material. However, a large quantity of ammonium chloride sometimes remains in the material obtained even after the heat treatment. In such cases, it is sometimes possible to remove the ammonium chloride by washing with water or a suitable aqueous solution to improve the visible light responsiveness of the visible light responsive type material.

In this case, washing the material obtained by heating with water or an aqueous solution is desirably conducted so that either the pH of the water or aqueous solution separated from the material following washing is 3.5 or greater (pH 3.5-7), or the quantity of chlorine ions (when titanium chloride is employed as the starting material of the hydrolysis product) or quantity of sulfuric acid ions (when titanium sulfate is employed as the starting material of the hydrolysis product) contained in the water or aqueous solution separated from the material following washing decreases.

Any of the visible light responsive optical materials described in the present *Specification* may be employed in the present invention. However, since few or no impurities are desirable with use in the human body, the use of a visible light responsive material obtained by baking a starting material in the form of titanium tetrachloride followed by washing with water is desirable.

The visible light responsive material is usually employed in the form of a powder. The granularity of the powder obtained can be adjusted by comminution or the like.

Silicon, aluminum, tin, zirconium, antimony, phosphorus, platinum, gold, silver, copper, iron, niobium, tungsten, tantalum, and other elements and compounds containing them can be coated on the surface of and/or carried within, or used to dope, the visible light responsive material as necessary. However, when used as a bleaching agent for teeth, the elements that are added are selected based on their effects on teeth.

The bleaching composition of the present invention comprises the above-described visible light responsive photocatalyst and peroxide. Examples of peroxides are hydrogen peroxide and urea peroxide. The concentration of the visible light responsive photocatalyst employed in the bleaching composition of the present invention ranges from 0.1 to 90 percent, desirably from 0.1 to 40 percent, and preferably from 0.1 to 6 percent. The concentration of the peroxide varies with the type of peroxide; for the example of hydrogen peroxide, the concentration ranges from 0.01 to 35 percent, desirably from 0.1 to 15 percent, and preferably from 0.1 to 4 percent. For the example of urea peroxide, the concentration ranges from 0.1 to 50 percent, desirably from 0.5 to 39 percent, and preferably from 15 to 25 percent.

The bleaching composition of the present invention may be employed in the form of a solution, gel, or the like. In addition to the above-described visible light responsive photocatalyst and peroxide, other suitable known components such as gelling agents, buffers, water, solvents, fragrance materials, and stabilizers may be incorporated.

For example, the bleaching composition of the present invention may be employed to bleach teeth, as well as suitably employed to bleach other products.

The method of bleaching teeth of the present invention comprises coating the bleaching composition of the present invention on the surface of a tooth and irradiating the coating with light having a component with a wavelength of greater than or equal to 420 nm. The bleaching composition can be applied to the tooth surface with a knife, brush, or the like, or by affixing a base material (preferably one transmitting light) that has been precoated or impregnated with the bleaching composition.

So as to achieve a substantive effect at the contact surface with the tooth, the quantity of bleaching composition applied to the surface of the tooth should be of a thickness that does not prevent the transmission of light; the effect does not improve with the thickness of the application.

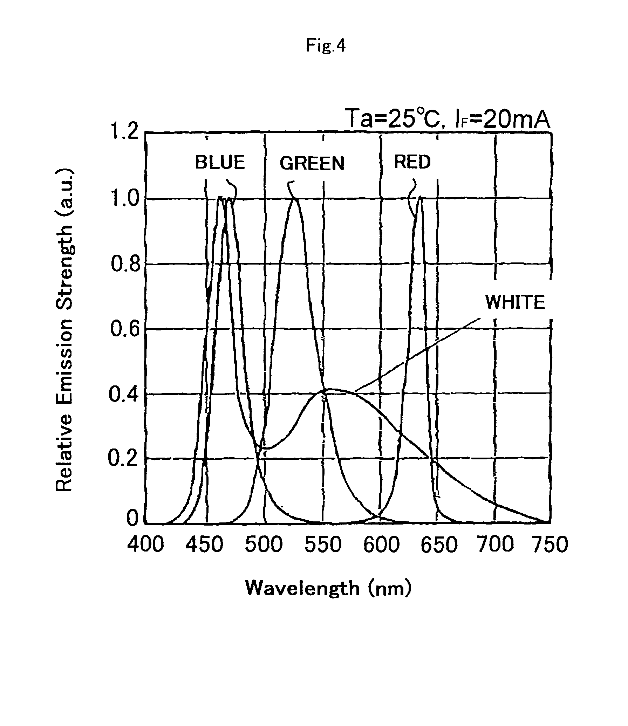

The light that is irradiated comprises a component with a wavelength of greater than or equal to 420 nm, and is preferably light comprising at least a component with a wavelength of from 420 to 600 nm. For example, light comprising at least a component with a wavelength of from 450 to 600 may be employed. For example, the light comprising a component with a wavelength of greater than or equal to 420 nm may be plasma light or light from a photopolymerizing device, light-emitting diode, or halogen lamp. The photopolymerizing device may be a photopolymerizing device for visible light polymerization employed in the curing of dental resins. The light-emitting diode may either have some light emission wavelengths greater than or equal to 420 nm in the visible light range, or may have light emission wavelengths restricted to within the visible light range. Examples of such light-emitting diodes are violet light-emitting diodes, blue light-emitting diodes, green light-emitting diodes, yellow light-emitting diodes, and white light-emitting diodes. Violet light-emitting diodes have light emission wavelengths from the ultraviolet range to the visible light range. Blue light-emitting diodes, green light-emitting diodes, yellow light-emitting diodes, and white light-emitting diodes have light emission wavelengths only in the visible light range. Fig. 4 shows the light emission spectra of blue light-emitting diodes (BLUE), green light-emitting diodes (GREEN), and white light-emitting diodes (WHITE).

For example, the device described in Japanese Unexamined Patent Publication (KOKAI) No. 2000-217844 may be employed as a dental light radiating device comprising a light-emitting diode. This dental light radiating device comprises multiple light-emitting diodes that are arranged in a curved manner in front of a row of teeth to uniformly irradiate and bleach an entire row of teeth with light.

The duration of the period of irradiation with light in the bleaching method of the present invention is suitably determined based on the makeup of the bleaching composition employed, the type and intensity of the light source, the discoloration of the teeth being bleached, and the degree of bleaching desired. The duration is usually less than or equal to 30 min, desirably less than or equal to 20 min, and preferably less than or equal to 10 min.

Following irradiation with light, the coated composition can be washed away with water or the like.

### Embodiments

Embodiments of the present invention are given below; however, the present invention is not limited thereto.

### Embodiment 1

Dental Bleaching Test

A 20 mg quantity of the powder prepared in Reference Example 3 was added to 0.5 g of a commercial dental bleaching gel (containing 21 percent urea peroxide), the components were thoroughly kneaded, and the mixture was uniformly applied to a thickness of 1 mm on teeth. Following the application, the coating was irradiated five times with light having a component of greater than or equal to 420 nm for a period of 1 minute each time with a commercial photopolymerization device (Apollo 95E made by DMD). Coloration of the teeth prior to and after treatment was measured with a VITA shade guard (reference colors); the results are given in Table 1. Results equivalent to those shown in Table 1 were obtained when irradiation with light comprising a component of greater than or equal to 420 nm was conducted continuously for 5 minutes with a D-LUX10 made by Dentrade as photopolymerization device.

**Comparative Example 1**

With the exception that commercially available ultrafine particulate titanium oxide powder (ST-01, made by Ishihara Sangyo) was employed instead of the powder prepared in Reference Example 3, a bleaching test was conducted in the same manner as in Embodiment 1. The results are given in Table 1.

**Comparative Example 2**

With the exception that commercially available photocatalytic titanium oxide powder P-25 (made by Degussa) was employed instead of the powder prepared in Reference Example 3, a bleaching test was conducted in the same manner as in Embodiment 1. The results are given in Table 1.

Table 1:

| Change in Tooth Coloration | | |
|---|---|---|
| | Before light irradiation | After light irradiation |
| Embodiment 1 | C4 | C1 |
| Comparative Example 1 | C4 | C4 |
| Comparative Example 2 | C4 | C4 |
| Embodiment 2 (blue LED) | C4 | C1 |
| Embodiment 3 (green LED) | C4 | C2 |
| Comparative Example 3 (blue LED) | C4 | C4 |
| Comparative Example 4 (green LED) | C4 | C4 |

**Embodiment 2 (Light-Emitting Diode)**

The same test was conducted as in Embodiment 1 with the exception that the photopolymerization device employed as light source was replaced with a blue light-emitting diode.
The blue light-emitting diode comprised one row of four blue LEDs (model NSSB450, made by Nichia Kagaku Kogyo (K.K.)) illuminated by applying 3.7 V to each light-emitting diode. The light irradiation period was 20 min. The coloration of the teeth prior to and after light irradiation was measured in the same manner as in Embodiment 1. The results are given in Table 1.

**Embodiment 3 (Light-Emitting Diode)**

The same test was conducted as in Embodiment 1 with the exception that the photopolymerization device employed as light source was replaced with a green light-emitting diode.
The green light-emitting diode comprised one row of four green LEDs (model NSSG450, made by Nichia Kagaku Kogyo (K.K.)) illuminated by applying 3.7 V to each light-emitting diode. The light irradiation period was 20 min. The coloration of the teeth prior to and after light irradiation was measured in the same manner as in Embodiment 1. The results are given in Table 1.

**Comparative Example 3**

The same bleaching test was conducted as in Embodiment 2 with the exception that commercially available ultrafine particulate titanium oxide powder (ST-01, made by Ishihara Sangyo) was employed in place of the powder prepared in Reference Example 3. The results are given in Table 1.

**Comparative Example 4**

The same bleaching test was conducted as in Embodiment 3 with the exception that commercially available ultrafine particulate titanium oxide powder (ST-01, made by Ishihara Sangyo) was employed in place of the powder prepared in Reference Example 3. The results are given in Table 1.

**Embodiment 4 (Light-Emitting Diode)**

Bleaching tests were conducted using the powders prepared in Reference Examples 4, 5, and 6 instead of the powder prepared in Reference Example 3 in Embodiments 1 and 2. The results are given in Table 2.

Table 2:

| Change in Tooth Coloration | | |
|---|---|---|
| Powder + Light Source | Before light irradiation | After light irradiation |
| Reference Example 4 + Photopolymerization Device | C4 | C1 |
| Reference Example 4 + Blue LED | C4 | C1 |
| Reference Example 5 + Photopolymerization Device | C4 | C1 |
| Reference Example 5 + Blue LED | C4 | C1 |
| Reference Example 6 + Photopolymerization Device | C4 | C2 |
| Reference Example 6 + Blue LED | C4 | C2 |

**Embodiment 5**

Hydroxy apatite (HAp) powder prepared in-house was molded by uniaxial pressing and baked for 2 hours at 1,150°C to obtain sintered members. A sintered member with a diameter of about 10 mm was soaked for 8 weeks in a dark location in a 5,000 ppm aqueous solution of tetracycline (Tec). Prior to bleaching, images of the surface of the HAp sintered member were picked up with a scanner (Epson GT-8700F). The center portion of the colored apatite was measured 10 times with Adobe Photoshop (registered trademark) for each image and the average thereof was adopted as the color measurement value. The L*, a*, and b* of each of the colored apatites were evaluated using the L*, a*, and b* color system to obtain measurement color specifications. Next, 6.66 weight percent of the visible light responsive material prepared in Reference Example 3, 4 weight percent of hydrogen peroxide, and 26.66 weight percent of a thickener in the form of silicic anhydride were mixed with a boric acid standard buffer solution to obtain a paste which was employed as a hydrogen peroxide aqueous solution bleaching agent containing visible light responsive material. An appropriate quantity of bleaching agent was coated onto the surface of the discolored HAp sintered member and then irradiated with blue light and green light from an LED light-emitting device at room temperature. Following 2 hours of irradiation, the bleaching agent was removed, water washing was conducted, and the change in color was checked by the same method. As a control, the above bleaching agent was applied without irradiation with light, washed with water 2 minutes later, and checked by the same method.

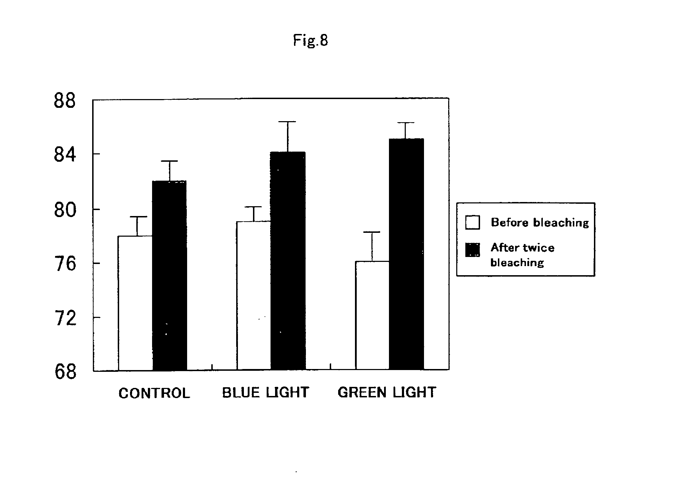

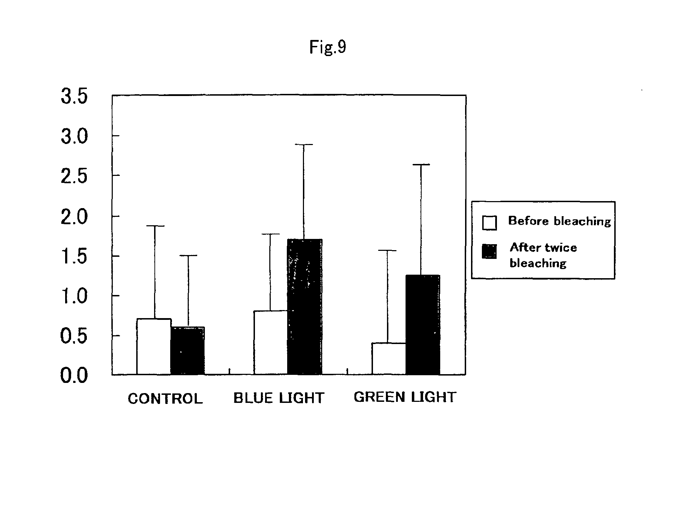

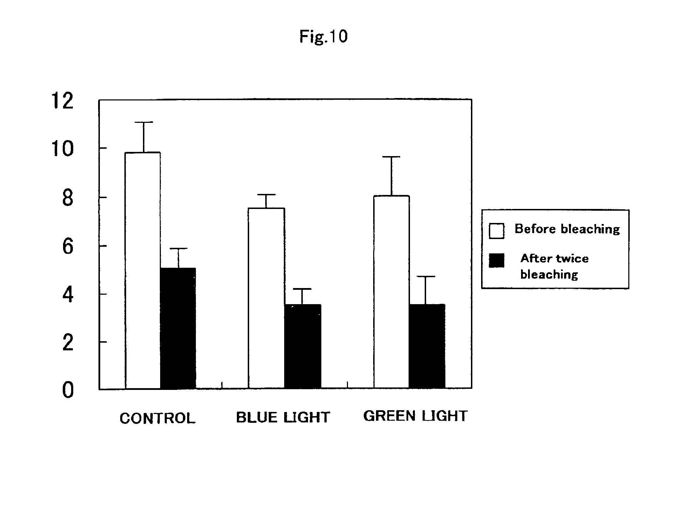

The HAp sintered member obtained had a relative density of 95 percent and was suitable for use as an enamel whitening model. Figs. 8, 9, and 10 give the L*, a*, and b* color system measurement results before and after bleaching of the tetracycline discolored apatite measured from images picked up by scanner.

All of the L* values increased over those prior to bleaching, indicating an increase in brightness. The average L* value with green light irradiation increased from 76.25 to 84.50, exhibiting the greatest level of bleaching.

The a* value increased with blue and green light irradiation, but there was no change in the control.

The b* value decreased in all cases, indicating a reduction in yellowing. However, no significant difference was observed relative to the control for either blue or green light irradiation. This indicated that the bleaching effect of the bleaching agent containing visible light responsive material was heightened by irradiation with green or blue light. In particular, the considerable bleaching effect that was achieved by irradiation with green light presented a marked difference from prior art bleaching agents.

**Reference example 1**

To pure ice water (two liters of water) were added 500 g of titanium tetrachloride (special grade, manufactured by Kanto Kagaku K.K.) and the mixture was stirred and dissolved, yielding a titanium tetrachloride aqueous solution. While stirring 200 g of this aqueous solution in a stirrer, about 50 mL of ammonia water (comprising 13 weight percent as $NH_3$) was added as rapidly as possible. The quantity of ammonia water added was adjusted to yield a final pH of about 8 in the aqueous solution. This converted the aqueous solution to a white slurry. After stirring for another 15 min, the mixture was filtered with a suction filter. The filtered precipitate was dispersed in 20 mL of ammonia water (containing 6 weight percent as $NH_3$) and stirred using a stirrer for about 20 hours. The mixture was then suction filtered again, yielding a white hydrolysis product.

The white hydrolysis product obtained was transferred to a crucible and heated to 400°C for 1 hour in air with an electric furnace, yielding a yellow product.

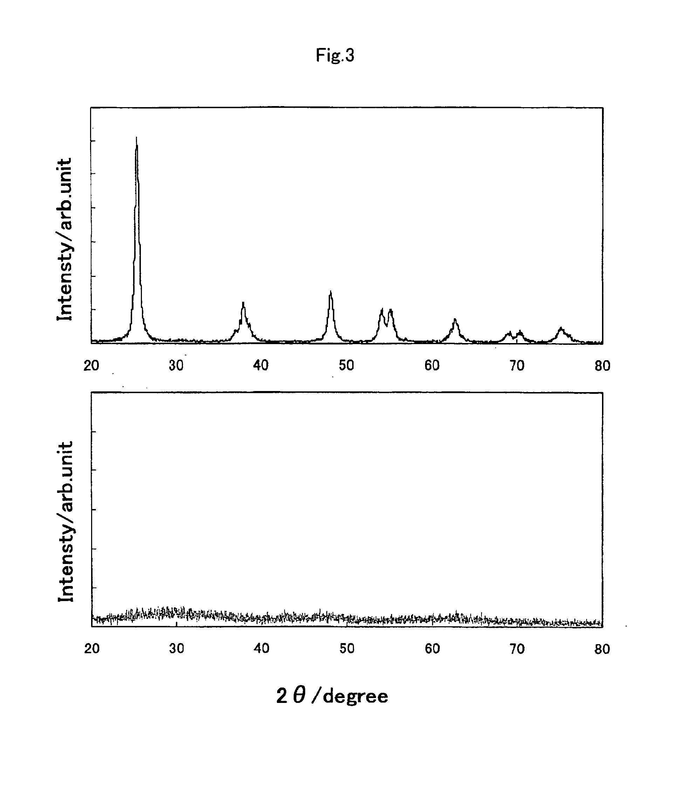

The results of XRD measurement of the obtained product are given in the upper segment of Fig. 3. In addition, the results of XRD measurement of the white hydrolysis product dried at 50°C are given in the lower segment of Fig. 3. From these results, it will be understood that the product obtained by drying the white hydrolysis product at 50°C was amorphous and the obtained product contained anatase titanium dioxide.

The diffuse reflectance spectra of the obtained product and the white hydrolysis product dried at 50°C obtained were measured under the following conditions with a Hitachi Autorecording Spectrophotometer (U-3210) equipped with integrating sphere:

| Scan speed | 120 nm/min, |
|---|---|
| Response | MEDIUM, |
| Band pass | 2.00 nm, and |
| Reference | barium sulfate. |

As a result, when the reflectance at 700 nm of the obtained product was defined as 100 percent, the reflectance thereof at 450 nm was 61 percent. By the contrast, when the reflectance at 700 nm of the product obtained by drying the white hydrolysis product at 50°C was defined as 100 percent, the reflectance thereof at 450 percent was 95 percent.

Further, the ESR spectrum of the obtained product was measured. Measurement was conducted at 77 K or ordinary temperature in vacuum (0.1 Torr or less). The measurement conditions were as follows:

[Basic Parameters]

Measurement temperature:  77 K or ordinary temperature
Field:  $324 \text{ mT} \pm 25 \text{ mT}$
Scan time:  4 min
Mod.:  0.1 mT
Receiver GAIN:  10-100 (measurement sensitivity)
Time constant:  0.1 sec
Light source:  500 W high-pressure mercury lamp
Filter:  L-42

[Sample Preparation]

Vacuum evacuation:     1 hr or more

[g Value Calculation]

$g = g_{mn} \times H_{mn}/(H_{mn} + \Delta H)$ based on $Mn^{2+}$ marker ($g_{mn}$ = 1.981 (third from the high magnetic field side)

$H_{mn}$: $Mn^{2+}$ marker     magnetic field
$\Delta H$:                  Amount of change in magnetic field from $H_{mn}$

Fig. 1 (measurement temperature 77 K) and Fig. 2 (measurement temperature ordinary temperature) show the ESR spectra in the dark in the upper segment, the ESR spectrum measured with irradiation with light through a filter (L-42) cutting light (obtained using a 500 W high-pressure mercury lamp) below 420 nm in the middle segment, and the ESR spectrum measured with irradiation of light employing a 500 W high-pressure mercury lamp without using a filter (L-42) cutting light below 420 nm is shown in the lower segment.

A comparison of the spectra of the upper and middle segments of Fig. 1 clearly reveals that in the middle segment spectrum, the main signal having a g value ranging from 2.004 to 2.007 and the two subsignals having g values ranging from 1.985 to 1.986 and 2.024 exhibited intensities stronger than in the spectrum in the upper segment. Further, a comparison of the spectra of the middle and lower segment spectra of Fig. 1 clearly reveals that neither the main signal having a g value ranging from 2.004 to 2.007 nor the two subsignals having g values ranging from 1.985 to 1.986 and 2.024 differed substantially even when light of less than 420 nm was included in the irradiating light.

Further, as shown in Fig. 2, the above-described three signals were observed in the ESR of the visible light responsive type material of Reference example 1 in darkness and with irradiation with light having a wavelength of 420 nm or more at ordinary temperature in air.

The main signal having a g value ranging from 2.004 to 2.007 and the two subsignals having g values ranging from 1.985 to 1.986 and 2.024 were not observed under any of the ESR measurement conditions in the white hydrolysis product dried at 50°C.

**Reference example 2**

A 3 g quantity of the powder obtained in Reference example 1 was suspended in 100 mL of pure water and stirred for 1 hr with a magnetic stirrer. The solution obtained was suction filtered. Sample remaining on the filter paper was again stirred in pure water and suction filtered. Filtration was repeated three times until the filtrate reached 6-7 on pH test paper.

The powder obtained was left standing for a day in a drier set to 110°C and dried, yielding the visible light responsive type material.

**Reference example 3**

A 23 kg quantity of titanium tetrachloride was gradually added to 207 kg of water having a temperature of 0°C held in a 300 L reaction vessel (permitting cooling and stirring). At that time, the temperature of the aqueous solution reached a maximum of 6°C. The titanium chloride was stirred for two days to prepare a transparent titanium tetrachloride aqueous solution. When 12.5 percent ammonia water was added dropwise while stirring the titanium tetrachloride aqueous solution that had been prepared, the solution gradually clouded over. The amount of ammonia water was adjusted so that the clouded solution reached pH 8.

The clouded solution was suction filtered. The white precipitate remaining on the filter paper weighed 131 kg. The white precipitate was dispersed in 200 kg of ammonia water (6 percent as $NH_3$), stirred for 24 hr, and suction filtered. The white precipitate following filtering weighed 108 kg. The white precipitate was placed in a forced ventilation shelf-type drier set to 50°C and dried for four days. Following drying, the sample weighed 17 kg.

A 1 kg quantity of the dried sample was placed in an alumina crucible (20 $\times$ 20 $\times$ 5 cm), the crucible was placed in a gas furnace, a thermocouple was placed on the surface of the sample, and the sample was sintered for 1 hr so that the sample temperature reached 400°C.

A 3 g quantity of the powder obtained was suspended in 100 mL of pure water and stirred for 1 hr with a magnetic stirrer. The solution obtained was suction filtered. Sample remaining on the filter paper was again stirred in pure water and suction filtered. Filtration was repeated three times until the filtrate reached 6-7 on pH test paper.

The powder obtained was left standing for a day in a drier set to 110°C and dried, yielding the visible light responsive type material.

**Reference example 4** (Method of manufacturing visible light responsive material from titanium sulfate (1))

Titanium sulfate (IV) aqueous solution (product name: Titanium sulfate (IV), made by Kanto Kagaku K.K. (Shika first grade, an aqueous solution comprising not less than 24 weight percent of titanium sulfate (IV)) was employed without alteration as titanium sulfate (IV) solution. While mixing 50 g of this aqueous solution with a stirrer, 58 mL of ammonia water (ammonia solution as provided:water =1:1) was added as rapidly as possible with a buret. When stirring was continued, clouding began and gradually increased viscosity. Ammonia water was further added to adjust the pH to 7 as indicated by universal test paper. When 24 hr had elapsed, filtration was conducted with a suction filter. The white product on the filter paper was stirred in ammonia water that had been adjusted to pH 11, filtering was repeated 8 times, and washing was conducted, yielding a white powder. The powder obtained was dried at 50°C, yielding sample powder. The BET surface area of the hydrolysis product obtained (sample powder) was 308.7 $m^2$/g. An 8 g quantity of the obtained sample powder was charged to a crucible, transferred to an electric furnace, and sintered for 60 min at 400°C, yielding 6.3 g of a clear yellow powder with a BET surface area of 89.4 $m^2$/g. The resulting sample powder was subjected to X-ray diffraction test (XRD) to reveal that it comprises anatase titanium oxide. X-ray photoelectron spectroscopy (XPS) of the resulting sample powder was measured with an X-ray photoelectron spectral analyzer unit (product name: Quantum 2000, made by Ulvacphi (K.K.)). As a result, the abundance ratio (O/Ti) of elemental oxygen and elemental titanium calculated from the area of the peak attributed to the 2p electron of titanium and the area of the peak attributed to the 1s electron of oxygen obtained shows that the powder has oxygen defects in the crystal structure. Due to this oxygen defects, it is believed that the powder shows clear or slight yellow color and visible light activity (photocatalytic activity).

The ESR spectrum of the powder obtained was measured. Measurement was conducted at 77 K in vacuum (0.1 Torr). The measurement conditions were identical to those in Reference Example 1.

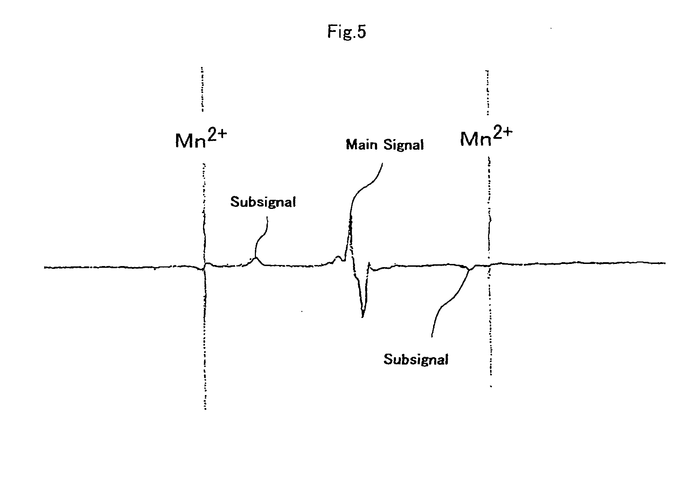

Fig. 5 shows the ESR spectra measured with irradiation with light through a filter (L-42) cutting out light (obtained using a 500 W high-pressure mercury lamp) below 420 nm (measurement temperature of 77 K). Although the ESR spectrum in darkness was also measured, essentially no signal was observed.

In the spectrum shown in Fig. 5, a main signal having a g value ranging from 2.004 to 2.007 and two subsignals having g values ranging from 1.985 to 1.986 and 2.024 were observed.

Neither a main signal having a g value ranging from 2.004 to 2.007 nor the two subsignals having g values ranging from 1.985 to 1.986 and from 1.985 to 2.024 were observed in product obtained by drying white hydrolysis product at 50°C under any of the ESR measurement conditions.

**Reference example 5** (Method of Manufacturing Visible Light Responsive Material from titanium sulfate (2))

A 50 g quantity of 24 percent titanium sulfate solution (Shika first grade, made by Kanto Kagaku) was added to 400 mL of distilled water and stirred with a magnetic stirrer. Concentrated ammonia water (28 percent, Kanto Kagaku, special grade) was added thereto and a neutralization reaction was conducted. Following the neutralization reaction, the mixture was adjusted to pH 7 and stirred for 15 min. Since the stirrer was sometimes no longer able to turn at that time, distilled water was added (200 mL). Fifteen minutes later, after stirring had been stopped, the mixture was left standing for some time and the supernatant was discarded. Filtration was conducted with a nutsche filter and the filtrate was washed with 2 L of ammonia water (5:95). This operation is a method wherein ammonia water was added when the mixture formed cakes on the filter paper. The mixture obtained was then dried for 24 hr at 60°C and sintered for 1 hr at 400°C, yielding the visible light responsive type material. NO oxidation activity of the resulted material was measured in accordance with the following Test Example.

**Test Example**

NO oxidation activity ($NO_x$ Elimination)

The visible light responsive type material prepared in Reference example 5 was placed in a Pyrex glass reaction vessel (internal diameter 160 mm, thickness 25 mm). Monochrome light with a half-width of 20 nm was irradiated with an irradiation unit made by Nippon Bunko employing a 300 W xenon lamp as light source.

A 1.5 L/min flow of 0 percent RH in humidity simulated polluted air (NO: 1 ppm) was continuously fed into the reaction vessel and the change in concentration of NO was monitored at the reaction discharge outlet. The NO concentration was measured by the chemoluminescence method using ozone. The $NO_x$ elimination rate was obtained from the cumulative value of the values monitored over 1 hr.

The $NO_x$ elimination activity of each material at 470nm was given in Table 3.

Table 3

| Wavelength (nm) | NO reduction (%) | $NO_2$ generation (%) | NOx elimination (%) |
|---|---|---|---|
| 570 | 1.2 | 0 | 1.2 |
| 520 | 9.8 | 1.2 | 8.6 |
| 470 | 21.5 | 4.3 | 17.2 |
| 420 | 22.2 | 5.5 | 16.6 |
| 360 | 28.5 | 10.3 | 18.2 |

The ESR spectrum of the material obtained was measured. The measurement was conducted in vacuum (0.1 Torr) at 77 K. The measurement conditions were identical to those in Reference example 1.

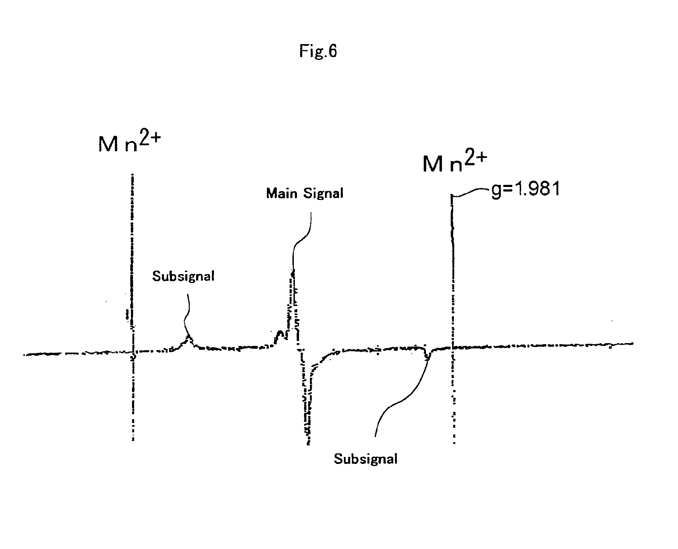

Fig. 6 (measurement temperature of 77 K) gives the ESR spectrum measured with irradiation by light passing through a filter (L-42) cutting out light (obtained using a 500 W high-pressure mercury lamp) below 420 nm.

A main signal having a g value ranging from 2.004-2.007 and two subsignals having g values ranging from 1.985 to 1.986 and 2.024 were observed in the spectrum of Fig. 6.

Neither a main signal having a g value ranging from 2.004-2.007 nor two subsignals having g values ranging from 1.985 to 1.986 and 2.024 were observed under any of the ESR measurement conditions in the product obtained by drying the white hydrolysis product at 50°C.

**Reference example 6** (Method of Manufacturing from Alkoxide)

To 200 g of pure water, 30 g of titanium isopropoxide were gradually added with stirring (with a molar ratio of water to titanium isopropoxide of about 10:1). The solution obtained was stirred for about 30 min and the precipitate (hydrolysis product) was recovered by filtration. The precipitate (hydrolysis product) was then suspended in pure water, stirred for one day, filtered, dried at 110°C, and sintered for 1 hr at 400°C. The white powder obtained was referred to as sample A.

With the exception that the precipitate (hydrolysis product) was suspended in ammonia water (ammonia concentration: 6 percent) and stirred for one day instead of being suspended in pure water and stirred for one day, a yellow powder referred to as sample B was obtained by the same operation as above.

The NO activity of samples A and B were measured by the above-described test method. The results are given in Table 4 below.

NO Activity

Table 4

| Sample A | | | |
|---|---|---|---|
| Wavelength (nm) | NO elimination (%) | $NO_2$ generation (%) | NOx elimination (%) |
| 570 | 0 | 0 | 0 |
| 520 | 0.7 | 0 | 0.7 |
| 470 | 1.4 | 0 | 1.4 |
| 420 | 1.9 | 0 | 1.9 |
| 360 | 16.0 | 2.5 | 13.5 |
| Sample B | | | |
| Wavelength (nm) | NO elimination (%) | $NO_2$ generation (%) | NOx elimination (%) |
| 570 | 1.0 | 0 | 1.0 |
| 520 | 4.7 | 0 | 4.7 |
| 470 | 15.6 | 3.4 | 12.2 |
| 420 | 16.4 | 4.6 | 11.8 |
| 360 | 19.1 | 10.0 | 9.1 |

From the results of Table 4, it will be understood that a material comprised of titanium oxide having visible light responsiveness was obtained by heat treating titanium oxide (titanium hydrolysis product) in the presence of ammonia.

The ESR spectra of the materials obtained were measured. The measurement was conducted at 77 K in vacuum (0.1 Torr). The measurement conditions were identical to those in Reference example 1.

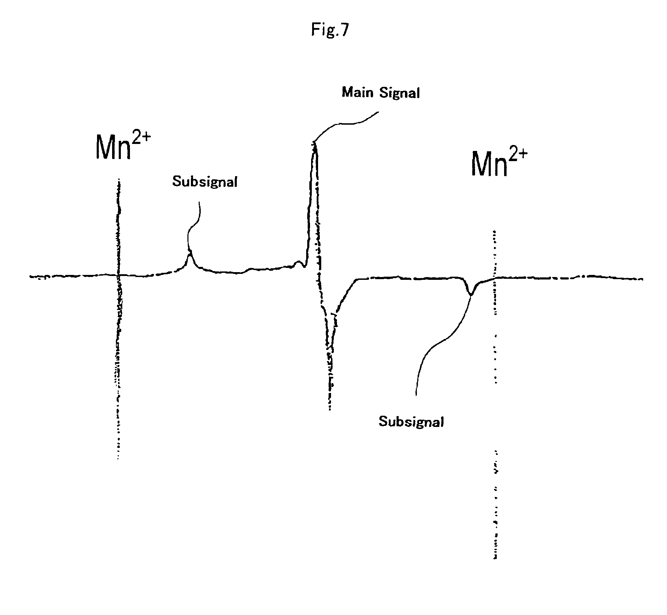

Fig. 7 (measurement temperature 77 K) gives the ESR spectra measured with irradiation by light passing through a filter (L-42) cutting out light (obtained using a 500 W high-pressure mercury lamp) below 420 nm.

A main signal having a g value ranging from 2.004-2.007 and two subsignals having g values ranging from 1.985 to 1.986 and from 1.985 to 2.024 were observed in the spectra of Fig. 7.

Neither a main signal having a g value ranging from 2.004-2.007 nor two subsignals having g values ranging from 1.985 to 1.986 and 2.024 were observed under any of the ESR measurement conditions in the product obtained by drying the white hydrolysis product at 50°C.

Industrial Applicability

The present invention provides a new bleaching agent and bleaching method employing photocatalysts that achieve desired effects more rapidly than prior art. They are particularly useful in bleaching teeth.

Specifically, the present invention utilizes a visible light responsive titanium oxide photocatalyst that reacts with visible light of greater than or equal to 420 nm, and in some cases, with visible light with a wavelength of greater than or equal to 450 nm, and does not require the utilization of the properties of either or both ultraviolet radiation or violet light. Accordingly, the desired effect is achieved more rapidly than in prior art. In this regard, the present invention differs fundamentally from the invention described in Japanese Unexamined Patent Publication (KOKAI) No. 2000-344640. The present invention is capable of effective bleaching by irradiation with light for a short period of several minutes and does not require coating with calcium phosphate.

Further, even within the visible light range, it has not been demonstrated that violet light with a wavelength of about 400 nm in the vicinity of ultraviolet radiation does not have a harmful effect on living organisms. The fact that bleaching is possible with visible light of greater than or equal to 420 nm, which can be considered to be almost completely safe, and sometimes with just visible light of a wavelength greater than or equal to 450 nm, can be considered highly useful and valuable in terms of safety for bleaching agents employed in various applications.

**Claims**

1. A bleaching composition comprising a peroxide and a photocatalyst (referred to hereinafter as a visible light responsive photocatalyst) activated by the effect of light having a wavelength of greater than or equal to 420 nm.

2. The bleaching composition according to claim 1, wherein the visible light responsive photocatalyst is activated by light having a wavelength of greater than or equal to 450 nm.

3. The bleaching composition according to claim 1 or 2, wherein the visible light responsive photocatalyst is a visible light responsive material in the form of titanium oxide comprising at least anatase titanium oxide, where in ESR measurement under irradiation by light having a wavelength of greater than or equal to 420 nm at 77 K under vacuum, a primary signal with a g value of from 2.004 to 2.007 and two secondary signals with g values of from 1.985 to 1.986 and 2.024 are detected, and these three signals are either only slightly detected or essentially undetected under vacuum at 77 K in the dark.

4. The bleaching composition according to any of claims 1 to 3, wherein the peroxide is hydrogen peroxide or urea peroxide.

5. The bleaching composition according to any of claims 1 to 4, wherein the bleaching composition is employed to bleach teeth.

6. A method of bleaching teeth comprising application of the composition according to any of claims 1 to 4 on the surface of a tooth and irradiating the resulting coating with light comprising a component with a wavelength of greater than or equal to 420 nm.

7. The method according to claim 6, wherein the light comprising a component with a wavelength of greater than or equal to 420 nm is plasma light or light generated by a photopolymerization device, light-emitting diode, or halogen lamp.

8. The method according to claim 7, wherein the light-emitting diode is a violet light-emitting diode, blue light-emitting diode, green light-emitting diode, yellow light-emitting diode, or white light-emitting diode.

Fig.1

Fig.2

Fig.3

Fig.4

Fig.5

Fig.6

Fig.7

Fig.8

Fig.9

Fig.10

## EP 1 375 635 A1

### INTERNATIONAL SEARCH REPORT

International application No.

PCT/JP02/01799

**A. CLASSIFICATION OF SUBJECT MATTER**

Int.Cl$^7$ C11D7/54, 7/20, A61K7/20

According to International Patent Classification (IPC) or to both national classification and IPC

**B. FIELDS SEARCHED**

Minimum documentation searched (classification system followed by classification symbols)

Int.Cl$^7$ C11D7/54, 7/20, A61K7/20

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2000-344640 A (Director General of Agency of Industrial Science and Technology), 12 December, 2000 (12.12.00), Claims; par. No. [0010] (Family: none) | 1-2,4-8<br>3 |
| X<br>Y | EP 1048291 A1 (Director General of Agency of Industrial Science and Technology), 02 November, 2000 (02.11.00), Claims<br>& US 6231343 B1 & WO 99/15143 A1<br>& JP 11-92351 A<br>Claims | 1-2,4-8<br>3 |
| Y<br>A | Kagaku Dai Jiten 2, reduced-size edition; 32nd print, Kyoritsu Shuppan Co., Ltd., 15 August, 1989 (15.08.89), Page 375, left column, lines 24 to 36 | 1-2,4-8<br>3 |

☒ Further documents are listed in the continuation of Box C. ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 22 April, 2002 (22.04.02) | 14 May, 2002 (14.05.02) |

| Name and mailing address of the ISA/<br>Japanese Patent Office | Authorized officer |
|---|---|
| Facsimile No. | Telephone No. |

Form PCT/ISA/210 (second sheet) (July 1998)

**INTERNATIONAL SEARCH REPORT**

| International application No. |
|---|
| PCT/JP02/01799 |

C (Continuation).   DOCUMENTS CONSIDERED TO BE RELEVANT

| Category* | Citation of document, with indication, where appropriate, of the relevant passages | Relevant to claim No. |
|---|---|---|
| Y<br>A | Shoichi YASUHO et al., "Kashiko Hanno Gata Hikari Shokubai no Sosei to sono Shuho(2) Ion Chunyu Ho oyobi Magnetron Sputtering Jochaku Ho no Oyo", Kogyo Zairyo, 01 June, 2000 (01.06.00), Vol.48, No.6, pages 32 to 36 | 1-2,4-8<br>3 |
| Y<br>A | Shoichi YASUHO, "Kinzoku Ion Chunyu niyoru Sanka Titanium Hikari Shokubai no Kashikoka", Annual reports, Research Center for Photoenergetics of Organic Materials, Osaka University, edition 1997, 1998.03, pages 43 to 44 | 1-2,4-8<br>3 |
| Y | WO 00/10706 A1  (Yugengaisha Kankyo Device Kenkyusho),<br>02 March, 2000 (02.03.00),<br>Claims<br>& EP 1125636 A1          & JP 2001-212457 A<br>Claims | 1-8 |
| Y | JP 62-207718 A  (Taki Chemical Co., Ltd.),<br>12 September, 1987 (12.09.87),<br>Claims<br>(Family: none) | 1-8 |
| Y | US 5011674 A  (Sakai Chemical Industry Co., Ltd.),<br>30 April, 1991 (30.04.91),<br>Claims<br>& JP 1-301518 A<br>Claims | 1-8 |

Form PCT/ISA/210 (continuation of second sheet) (July 1998)